# FASCICULE DE BREVET EUROPEEN

(11) **EP 3 313 822 B1**
(45) Date de publication et mention de la délivrance du brevet: **14.09.2022**
(21) Numéro de dépôt: 16733039.8
(22) Date de dépôt: 23.06.2016
(51) Int. Cl.: C07D 233/34

(54) **PROCÉDÉ DE SYNTHÈSE D'OXIMES AROMATIQUES**
VERFAHREN ZUR SYNTHESE VON AROMATISCHEN OXIMEN
METHOD FOR SYNTHESIZING AROMATIC OXIMES

(30) Priorité: 24.06.2015 FR 1555821
(43) Date de publication de la demande: 02.05.2018
(73) Titulaire: Compagnie Générale des Etablissements Michelin, 63000 Clermont-Ferrand (FR)
(72) Inventeur: COUTURIER, Jean-Luc, 69006 Lyon (FR); LEDUC, Philippe, 69590 Larajasse (FR); FLEURY, Etienne, 63040 Clermont-Ferrand Cedex 09 (FR); SALIT, Anne-Frédérique, 63040 Clermont-Ferrand Cedex 09 (FR); UGOLNIKOV, Oleg, 63040 Clermont-Ferrand Cedex 09 (FR); IVANOV, Sergey, 63040 Clermont-Ferrand Cedex 09 (FR)
(74) Mandataire: Gandon-Pain, Sylvie
(86) Numéro de dépôt international: PCT/EP2016/064491
(87) Numéro de publication internationale: WO 2016/207261

(56) Documents cités:
- WO-A1-2012/007684
- CN-A- 101 270 071

## Description

La présente invention concerne un procédé de synthèse de molécules comprenant une fonction oxime.

Une oxime peut se transformer aisément par oxydation en un oxyde de nitrile, connu pour réagir avec des insaturations, notamment carbone-carbone, qui seraient présentes sur un polymère pour former une liaison covalente avec ledit polymère. Dès lors, lesdites molécules, pour peu qu'elles portent par ailleurs une autre fonction, par exemple interactive vis-à-vis d'une charge apparaissent comme des intermédiaires réactionnels intéressants pour greffer des fonctions sur un polymère.

En effet, dans le domaine industriel, des mélanges de polymères avec des charges sont souvent utilisés. Pour que de tels mélanges présentent de bonnes propriétés, on recherche en permanence des moyens pour améliorer la dispersion des charges au sein des polymères. L'un des moyens pour parvenir à ce résultat est l'utilisation d'agents de couplage capables d'établir des interactions entre le polymère et la charge.

Compte-tenu de l'intérêt de ces molécules, l'un des enjeux est de pouvoir disposer d'oximes, le cas échéant portant une fonction interactive vis-à-vis d'une charge, dont la préparation requiert le moins d'étapes possibles, sans pour autant sacrifier la pureté des oximes ni leur rendement d'obtention. La multiplication des étapes a un impact sur le rendement global du procédé de synthèse du composé désiré mais aussi sur les coûts d'investissement et les coûts de production associés. Par ailleurs, une autre préoccupation dans la synthèse des oximes est de minimiser le nombre et la quantité de solvants utilisés dans le procédé de préparation, afin de faciliter le traitement des effluents, de réduire les coûts d'investissement et de production associés, bien sûr sans affecter ni le rendement en oximes, ni la pureté des oximes.

La présente invention a pour objet de proposer une solution permettant de résoudre l'ensemble des problèmes mentionnés ci-dessus.

Selon l'invention, un procédé de synthèse d'un composé de formule (I) : 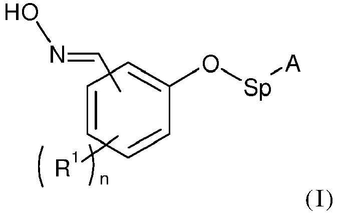 dans laquelle :
- A est le groupe (Va) 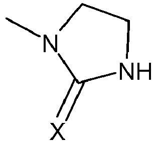 (Va), dans laquelle X est choisi parmi l'atome d'oxygène et l'atome de soufre ;
- R¹ représente un groupe alkyle en C₁-C₁₂ ou OR', R' étant un groupe alkyle en C₁-C₁₂ ;
- n est un nombre entier allant de 0 à 4 ;
- Sp représente une chaîne alkylène linéaire ou ramifiée en C1-C24, éventuellement interrompue par un ou plusieurs atomes d'azote ou d'oxygène,
   comprend deux étapes successives (b1) et (b2) selon une synthèse monotope :
      - (b1) la réaction d'un composé de formule (II) : 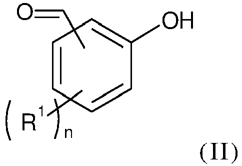
   avec un composé de formule (III) : Z-Sp-A (III) dans laquelle Z représente un groupe nucléofuge ;
   en présence :
      - d'au moins un solvant polaire ;
      - d'au moins une base choisie parmi les carbonates alcalins ;
   à une température T₁ allant de 70 à 150°C,
   pour former un éther ;
      - (b2) : la réaction dudit éther avec une solution aqueuse d'hydroxylamine à une température T₂ allant de 30 à 70°C.

Les étapes (b1) et (b2) sont « one pot » (procédé de synthèse monotope en deux étapes), c'est-à-dire sans isolation de l'éther intermédiaire.

On entend par solvant polaire au sens de la présente invention un solvant présentant une constante diélectrique supérieure à 2,2.

De préférence, l'étape (b2) est suivie d'une étape (c) de récupération du composé de formule (I).

On entend par groupe nucléofuge au sens de la présente invention un groupe partant qui emporte son doublet de liaison.

On entend par chaîne carbonée au sens de la présente invention une chaîne comprenant un ou plusieurs atomes de carbone.

Il est précisé que les expressions « de... à... » utilisées dans la présente description doivent s'entendre comme incluant chacune des bornes mentionnées.

Le procédé selon l'invention permet de synthétiser le composé de formule (I) à partir du composé de formule (II) selon une synthèse monotope en deux étapes. Selon la demande de brevet WO 2012/007684, le composé de formule (I) est également obtenu à partir du composé de formule (II) mais le composé intermédiaire éther est isolé et purifié.

D'autres avantages et caractéristiques de l'invention apparaitront plus clairement à l'examen de la description détaillée.

Le procédé selon l'invention permet la synthèse du composé de formule (I) mentionnée ci-dessus.

On entend par groupe carboné au sens de la présente invention un groupe comprenant un ou plusieurs atomes de carbone.

A est le groupe de formule (Va) : 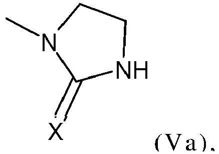 dans lequel X est choisi parmi l'atome d'oxygène et l'atome de soufre, de préférence l'atome d'oxygène.

Le groupe Sp est un groupe espaceur qui permet de relier l'atome d'oxygène au groupe A tel que la formule du composé (I) l'indique.

Le groupe Sp est une chaîne alkylène linéaire ou ramifiée en C₁-C₂₄, préférentiellement en C₁-C₁₀, éventuellement interrompue par un ou plusieurs atomes d'azote ou d'oxygène, plus préférentiellement une chaîne alkylène linéaire en C₁-C₆.

De préférence, le groupe Sp contient un motif choisi parmi-(CH₂)_{y1}-, -[NH-(CH₂)_{y2}]ₓ₁- et -[O-(CH₂)_{y3}]ₓ₂-, y₁, y₂ et y₃ représentant, indépendamment, un nombre entier allant de 1 à 6, et x₁ et x₂ représentant, indépendamment, un nombre entier allant de 1 à 4.

R¹ représente un groupe alkyle en C₁-C₁₂, plus préférentiellement en C₁-C₆, encore plus préférentiellement en C₁-C₄, ou OR', R' étant un groupe alkyle en C₁-C₁₂, plus préférentiellement en C₁-C₆, encore plus préférentiellement en C₁-C₄, encore plus préférentiellement un groupe méthyle, éthyle ou un groupe OCH3.

Avantageusement, n est un nombre entier allant de 0 à 3.

Comme expliqué précédemment, le procédé selon l'invention comprend une étape (b1) de réaction d'un composé de formule (II), tel que mentionnée ci-dessus, avec un composé de formule (III) : Z-Sp-A (III), telle que mentionnée ci-dessus.

De manière préférée, le groupe Z est choisi parmi le chlore, le brome, l'iode, le groupe mésylate, le groupe tosylate, le groupe acétate et le groupe trifluorométhylsulfonate.

Ladite étape (b1) du procédé selon l'invention est réalisée en présence d'au moins un solvant polaire, et d'au moins une base choisie parmi les carbonates alcalins, à une température T₁ allant de 70 à 150°C.

Avantageusement, le solvant polaire est un solvant polaire miscible dans l'eau, préférentiellement un solvant protique.

Le diméthylformamide (DMF), le diméthylsulfoxyde (DMSO), la 1,3-diméthyl-2-imidazolidinone (DMI), la 1,3-diméthyl-3,4,5,6-tetrahydro-2(1H)-pyrimidinone (DMPU), l'isopropanol, l'acétonitrile, l'éthanol, le n-butanol et le n-propanol sont des exemples de solvants utilisables dans le procédé selon l'invention.

Selon un mode de réalisation particulier de l'invention, le solvant protique est alcoolique.

De préférence, le composé de formule (II) représente de 5 à 40% en poids, de préférence de 10 à 30% en poids, par rapport au poids du solvant.

Avantageusement, il est possible d'ajouter :
- un ou plusieurs catalyseurs choisis parmi un catalyseur de type sel d'argent (I), un catalyseur de transfert de phase de type ammonium quaternaire, et leurs mélanges ;
- un ou plusieurs liquides ioniques.

Préférentiellement, la base est choisie parmi le carbonate de potassium.

Selon un mode de réalisation particulier de l'invention, la quantité de base est de 1,5 à 8 équivalents, de préférence de 2 à 6 équivalents, par rapport à la quantité de composé de formule (II).

Comme expliqué précédemment, l'étape (b1) du procédé selon l'invention est réalisée à une température T₁ allant de 70 à 150°C.

De préférence, la température T₁ est une température allant de 70 à 120°C, plus préférentiellement de 80 à 110°C.

Comme expliqué précédemment, l'étape (b1) du procédé selon l'invention est suivie de l'étape (b2) de l'ajout dans le milieu réactionnel contenant ledit éther d'une solution aqueuse d'hydroxylamine à une température T₂ allant de 30 à 70°C.

De préférence, l'ajout de la solution aqueuse d'hydroxylamine est réalisé lorsque la conversion du composé de formule (II) est d'au moins 70%.

Avantageusement, la température T₂ varie de 40 à 60°C.

Préférentiellement, le procédé selon l'invention comprend une étape (c) de récupération, telle que mentionnée ci-avant, du composé de formule (I).

De manière préférée, le composé de formule (I) est récupéré par précipitation à l'eau, suivie éventuellement d'un lavage à l'eau.

Selon un mode particulier de l'invention, le procédé selon l'invention comprend une étape (a1) de fabrication du composé de formule (II), préalable à l'étape (b1), par mise en réaction d'un composé de formule (X) : 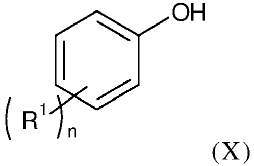 avec un agent de formylation formé in situ ou non et au moins un acide de Lewis, en présence d'au moins un solvant organique.

Préférentiellement, l'acide de Lewis est choisi parmi TiCl₄ et SnCl₄, plus préférentiellement TiCl₄.

De préférence, la quantité d'acide de Lewis est de 0,5 à 4 équivalents, plus préférentiellement de 1 à 3 équivalents, par rapport à la quantité de composé de formule (X).

Selon un mode de réalisation particulier de l'invention, le solvant organique est un solvant chloré, préférentiellement choisi parmi le dichlorométhane, le chloroforme et le 1,2-dichloroéthane, plus préférentiellement le dichlorométhane.

Comme expliqué précédemment, le composé de formule (X) réagit avec un agent de formylation formé in situ ou non, selon une réaction de formylation. On peut utiliser tout type d'agent de formylation classiquement employé dans les réactions de formylation.

Préférentiellement, l'agent de formylation est choisi parmi le dichlorométhyl-méthyléther et le dichlorométhyl-éthyléther, plus préférentiellement le dichlorométhyl-méthyléther.

Avantageusement, l'agent formé in situ est obtenu par réaction du formiate de méthyle ou d'éthyle avec un agent de chloration, préférentiellement le pentachlorure de phosphore.

De préférence, le composé de formule (X) représente de 5 à 15% en poids par rapport au poids du solvant.

Selon un mode de réalisation particulier de l'invention, le procédé selon l'invention comprend une étape (a2) de fabrication du composé de formule (III), préalable à l'étape (bl), par mise en réaction d'un composé de formule (XI) : HO-Sp-A (XI) avec un agent permettant la formation du groupe nucléofuge Z.

De manière préférée, ledit agent est le chlorure de thionyle.

De préférence, l'étape (a2) est réalisée en l'absence ou en présence d'au moins un solvant, préférentiellement un solvant chloré, plus préférentiellement le dichlorométhane.

Avantageusement, l'étape (a2) est immédiatement suivie d'une étape (a3) de récupération du composé de formule (III), préférentiellement par purification au toluène, plus préférentiellement par cristallisation du composé de formule (III) dans le toluène. Le procédé selon l'invention permet la synthèse du composé de formule (I) tel que mentionnée ci-dessus. Ledit composé, porteur d'une fonction oxime, peut éventuellement être soumis à une réaction d'oxydation pour se transformer en un composé porteur d'une fonction oxyde de nitrile, tel que décrit par exemple dans la demande WO 2012/007684.

La présente invention est en outre illustrée par les exemples non limitatifs suivants.

### EXEMPLES

L'analyse structurale ainsi que la détermination des puretés molaires des molécules de synthèse sont réalisées par une analyse RMN. Les spectres sont acquis sur un spectromètre Avance 500 MHz BRUKER équipé d'une sonde "large bande" BBIz-grad 5 mm. L'expérience RMN ¹H quantitative, utilise une séquence simple impulsion 30° et un délai de répétition de 3 secondes entre chacune des 64 acquisitions. Les échantillons sont solubilisés dans le diméthylsulfoxide deutéré (DMSO). Ce solvant est également utilisé pour le signal de lock. La calibration est réalisée sur le signal des protons du DMSO deutéré à 2,44 ppm par rapport à une référence TMS à 0 ppm. Le spectre RMN ¹H couplé aux expériences 2D HSQC ¹H/¹³C et HMBC ¹H/¹³C permettent la détermination structurale des molécules (cf tableaux d'attributions). Les quantifications molaires sont réalisées à partir du spectre RMN 1D ¹H quantitatif.

L'analyse par spectrométrie de masse est réalisée en injection directe par un mode d'ionisation en electrospray (ID/ESI). Les analyses ont été réalisées sur un spectromètre HCT Bruker (débit 600 µL/min, Pression du gaz nébuliseur 10 psi, débit du gaz nébuliseur 4 L/min).

### Exemple 1 : Synthèse de l'oxime de 2-(2-(2-oxoimidazolidin-1-yl)éthoxy)benzaldéhyde

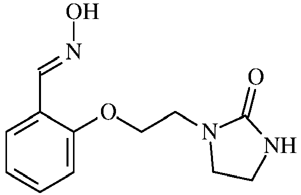

L'oxime de 2-(2-(2-oxoimidazolidin-1-yl)éthoxy)benzaldéhyde est synthétisée à partir du saliçaldéhyde et de la 1-(2-chloroéthyl)imidazolidin-2-one grâce à une synthèse monotope en deux étapes b1 et b2. Ladite oxime peut être préparée indépendamment selon les deux protocoles 1 et 2 indiqués ci-dessous.

### Protocole 1 (selon l'invention) :

Dans un réacteur de 1L, on charge 30 g de saliçaldéhyde (0,25 mol) et 200 g d'éthanol. On ajoute 172,8 g de carbonate de potassium (1,25 mol) et on chauffe à reflux. On ajoute ensuite, par portions et sur une période de 5 heures, 92,8 g de 1-(2-chloroéthyl)imidazolidin-2-one (0,63 mol). A la fin de l'ajout, on laisse réagir pendant 2 heures à reflux. On refroidit à 50°C, puis on ajoute, sur une période de 15 minutes, 24,8 g d'une solution aqueuse d'hydroxylamine à 50% (0,38 mol). On laisse réagir pendant 2 heures à 50°C. Le mélange réactionnel est concentré jusqu'à un volume de 50 mL, puis on ajoute, à température ambiante, 500 mL d'eau. Le précipité obtenu est filtré, lavé à l'eau puis au pentane, et séché sous vide.

Un solide blanc (40,5 g, rendement massique de 65%) avec un point de fusion de 88°C est obtenu.

### Protocole 2 (selon l'invention) :

Dans un réacteur de 1L, on charge 30 g de saliçaldéhyde (0,25 mol) et 300 g d'acétonitrile. On ajoute 172,8 g de carbonate de potassium (1,25 mol) et on chauffe à reflux. On ajoute ensuite, par portions et sur une période de 6 heures, 109,5 g de 1-(2-chloroéthyl)imidazolidin-2-one (0,74 mol). A la fin de l'ajout, on laisse réagir pendant 2 heures à reflux. On refroidit à 50°C, puis on ajoute, sur une période de 15 minutes, 24,8 g d'une solution aqueuse d'hydroxylamine à 50% (0,38 mol). On laisse réagir pendant 2 heures à 50°C. Le mélange réactionnel est concentré, puis on ajoute, à température ambiante, 500 mL d'eau. Le précipité obtenu est filtré, lavé à l'eau puis au pentane, et séché sous vide.

Un solide blanc (41,8 g, rendement massique de 70%) avec un point de fusion de 88°C est obtenu.

### Exemple 2 : Synthèse de l'oxime de 2,4,6-triméthyl-3-(2-(2-oxoimidazolidin-1-yl)éthoxy)benzaldéhyde

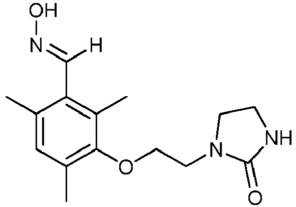

L'oxime de 2,4,6-triméthyl-3-(2-(2-oxoimidazolidin-1-yl)éthoxy)benzaldéhyde est préparée selon deux voies de synthèse. Selon une première voie, ladite oxime est synthétisée en quatre étapes, nommées étape a1, étape a2, étape b1 et étape b2. La seconde voie de synthèse diffère de la première voie en ce que les étapes b1 et b2 sont réalisées selon une synthèse monotope en deux étapes.

Pour chacune des étapes, les composés peuvent être synthétisés indépendamment selon plusieurs protocoles. L'étape a1 est réalisée selon divers protocoles 3 à 8, l'étape a2 selon divers protocoles 9 à 11, l'étape b1 selon divers protocoles 12 à 14, et l'étape b2 selon deux protocoles 15 et 16.

La synthèse monotope en deux étapes est réalisée selon divers protocoles 17 à 20.

### Etape a1 : préparation du 3-hydroxy-2,4,6-triméthylbenzaldéhyde

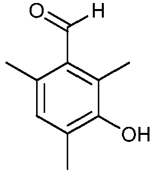

### Protocole 3 :

Ce composé peut être obtenu selon un protocole décrit dans la demande de brevet WO 2012/007684.

### Protocole 4 :

A une solution de TiCl₄ (10,44 g, 0,055 mol) dans le dichlorométhane (5 mL) refroidi à 12 °C, est ajoutée pendant 10 minutes une solution de mésitol (5,0 g, 0,037 mol) dans le dichlorométhane (15 mL). Après agitation pendant 5-10 minutes à 10-15°C, une solution de dichlorométhyl-méthyléther (6,75 g, 0,059 mol) dans le dichlorométhane (5 mL) est ajoutée sur une période de 10-15 minutes. Après 15 heures à température ambiante, un mélange de 100 mL d'eau et 50 g de glace est versé dans le milieu réactionnel. Après 30-40 minutes d'agitation, le brut réactionnel est filtré, lavé par l'eau (4 fois par 10 mL) et séché sous air.

Le produit cible (3,93 g) est obtenu avec un rendement massique de 65 %.

La pureté molaire estimée par RMN ¹H est supérieure à 90 %.

Chromatographie sur couches minces (CCM) : Rf = 0,87 (SiO₂ ; EtOAc ; révélation par UV et I₂), ou Rf = 0,35 (SiO₂ ; heptane : EtOAc = 3 : 1 ; révélation par UV et I₂).

### Protocole 5 :

A une solution de mésitol (6,0 g, 0,044 mol) dans le dichlorométhane (30 mL) (sous atmosphère inerte) à 2°C, est ajouté goutte à goutte du TiCl₄ (15,04 g, 0,079 mol) sur 15-20 minutes en maintenant la température du milieu réactionnel inférieure à 8°C. Après 5 minutes d'agitation à 6°C, le dichlorométhylméthyl éther (7,60 g, 0,066 mol) est ajouté pendant 35-40 minutes. Après 16 heures à température ambiante, un mélange de 75 mL d'eau et 50 g de glace est versé dans le milieu réactionnel. Apres 1 heure et 30 minutes d'agitation, le produit est filtré, lavé par l'eau (4 fois par 5 mL), et séché sous air.

Le produit cible (5,53 g, 0,034 mol) est obtenu avec un rendement massique de 76 %.

La pureté molaire estimée par RMN ¹H est supérieure à 98 %.

CCM : Rf = 0,87 (SiO₂ ; EtOAc ; révélation par UV et I₂), ou Rf = 0,35 (SiO₂ ; heptane : EtOAc = 3 : 1 ; révélation par UV et I₂). Protocole 6 :
A une solution de SnCl₄ (183,6 g, 0,705 mol) dans le dichlorométhane (250 mL) refroidie à 13°C, est ajoutée sur 20 minutes une solution de mésitol (40,0 g, 0,294 mol) dans le dichlorométhane (250 mL). Après 5 minutes d'agitation à 13°C, une solution de dichlorométhylméthyl éther (50,6 g, 0,441 mol) dans le dichlorométhane (100 mL) est ajoutée pendant 30 minutes. Le milieu réactionnel est agité pendant 15 heures à température ambiante.

Le milieu réactionnel est ensuite versé sur un mélange eau/glace (800 mL d'eau et 0,7 kg de glace). Après une heure d'agitation, la phase organique est séparée. La phase aqueuse est extraite par CH₂Cl₂ (2 fois par 100 mL). Les phases organiques réunies sont lavées par l'eau (2 fois par 100 mL), séchées par Na₂SO₄ et concentrées sous pression réduite (10 mbar, 23°C) pour conduire à 53 g d'une huile.

Une solution aqueuse de diméthyle amine (500 mL, 40% dans l'eau) est ajoutée au brut réactionnel. Le mélange est chauffé à 60°C et agité à cette température pendant 2,5 heures. De l'eau (1,0 L) est ajoutée et la phase aqueuse est extraite par le dichlorométhane (4 fois par 100 mL). Les phases organiques rassemblées sont lavées par une solution de HCl/eau (1 : 2) (trois fois par 100 mL) et par l'eau (trois fois par 100 mL). Après concentration sous pression réduite (150 mbar, 23°C), de l'éther de pétrole 40/60 (100 mL) est ajouté. La solution est refroidie jusqu'à -18°C pendant 15 heures. Les cristaux obtenus sont filtrés et lavés par un mélange de dichlorométhane (5 mL) et d'éther de pétrole 40/60 (15 mL), puis par de l'éther de pétrole 40/60 (deux fois par 20 mL), et enfin séchés sous air.

Le solide (20,02 g) est obtenu avec un rendement massique de 42 %.

Après concentration sous pression réduite du filtrat (60 mbar, 23°C), de l'éther de pétrole 40/60 (70 mL) y est ajouté. La solution est refroidie à -18 °C pendant 6 heures. Les cristaux obtenus sont filtrés sont lavés par l'éther de pétrole 40/60 (deux fois par 10 mL), et séchés sous air.

La seconde fraction de produit (3,02 g) est obtenue avec un rendement de 6,3 %.

Une purification par trituration est réalisée selon le protocole indiqué ci-dessous.

Les deux fractions de solides sont réunies et solubilisées dans un mélange de dichlorométhane (150 mL) et d'éther de pétrole 40/60 (100 mL). La solution est refroidie à -18°C pendant 4 à 6 heures. Les cristaux sont filtrés, lavés par l'éther de pétrole 40/60 (deux fois par 20 mL), et séchés sous air.

Le produit (11,9 g) est obtenu avec un rendement massique de 25 %.

La pureté molaire estimée par RMN ¹H est supérieure à 96 %.

CCM : Rf = 0,87 (SiO₂ ; EtOAc ; révélation par UV et I₂), ou Rf = 0,35 (SiO₂ ; heptane : EtOAc = 3 : 1 ; révélation par UV et I₂).

### Protocole 7 :

A du PCl₅ (91,7 g, 0,441 mol), sous atmosphère inerte, est ajouté à 22-29°C pendant 70 minutes du formiate d'éthyle (34,8 g, 0,470 mol). La réaction étant exothermique, la température du milieu peut atteindre 40-60 °C. Le milieu réactionnel est agité pendant 1 heure à température ambiante. Une fois le PCl₅ totalement solubilisé, le milieu réactionnel est agité 1-2 heures supplémentaires à température ambiante. L'agent de formylation ainsi formé est utilisé dans l'étape suivante sans purification supplémentaire.

A une solution de mésitol (40,0 g, 0,294 mol) dans le dichlorométhane (100 mL) à 11°C, est ajouté du TiCl₄ (105,0 g, 0,529 mol) sur 45 minutes. Après 5 minutes à 12-13°C, l'agent de formylation précédemment préparé est additionné sur 1,5-2,0 heures en maintenant la température entre 9 et 22°C. La suspension obtenue est agitée pendant 15 heures à température ambiante. Le milieu réactionnel est ensuite versé sur un mélange d'eau (500 mL) et de glace (500 g). Apres 15-20 minutes d'agitation, le précipité est filtré et lavé par l'eau (5 fois par 50 mL). Les cristaux obtenus sont séchés sous air.

Le produit cible (27,5 g, 0,170 mol) est obtenu avec un rendement massique de 58 %.

La pureté molaire estimée par RMN ¹H : 97 % mol.

CCM : Rf = 0,35 (SiO₂ ; heptane : EtOAc = 3 : 1 ; révélation par UV et I₂).

### Protocole 8 :

Dans un réacteur de 2 L muni d'un réfrigérant et purgé à l'azote, on charge 105,8 g de tétrachlorure de titane (0,56 mol). On refroidit à 15°C, et on coule, sur une période de 30 minutes, 40 g de mésitol (0,29 mol) dissous dans 400 g de dichlorométhane. En maintenant la température à 15-20°C, on coule ensuite, sur une période d'une heure, 40,6 g de dichlorométhyl-méthyléther (0,35 mol). A la fin de l'ajout, on laisse réagir pendant 3 heures à température ambiante. Le mélange réactionnel est transféré lentement dans un réacteur contenant 800 g d'eau et maintenu à une température inférieure à 20°C. Après 30 minutes sous agitation, on décante et on récupère la phase organique. La phase aqueuse est extraite deux fois avec 100 g de dichlorométhane. Les phases organiques rassemblées sont lavées avec 200 g d'eau, puis concentrées jusqu'à un volume de 100 mL. On rajoute 100 g de pentane, puis on refroidit à -15°C. Les cristaux obtenus sont lavés avec 50 g d'un mélange dichlorométhane-pentane 1/1 puis séchés sous vide.

Un solide blanc-beige (45,4 g, rendement massique de 94%) avec un point de fusion de 113°C est obtenu.

### Etape a2 : préparation de la 1-(2-chloroéthyl)imidazolidin-2-one

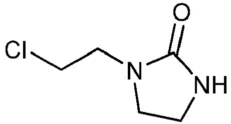

### Protocole 9 :

Ce composé peut être obtenu selon un protocole décrit dans la demande de brevet WO 2012/007684.

### Protocole 10 :

Sur de la 1-(2-hydroxyéthyl)imidazolidin-2-one (séché sous pression réduite à 60°C) (5,0 g, 38,42 mmol) est ajouté goutte à goutte du SOCl₂ (5,0 mL, 69,34 mmol) pendant 2-3 minutes. Le mélange est agité à 80°C (T_{bain}) pendant 1 heure et 30 minutes puis une heure et 30 minutes à 100°C (T_{bain}). Après évaporation de l'excès de SOCl₂ sous pression réduite (T_{bain} 50 °C, 8-9 mbar), le brut réactionnel (7,53 g, rendement 105,9 %) est obtenu sous la forme d'une huile jaune. De l'eau (10 mL) est ajoutée au brut réactionnel et le pH de la phase aqueuse est ajusté à 8 par addition de carbonate de potassium.

Apres cristallisation à température ambiante dans l'eau, le précipité est filtré et lavé par l'eau (2-3 mL). Les cristaux sont séchés sous air.

Le produit cible (2,87 g, 19,31 mol) est obtenu avec un rendement massique de 50 %.

La pureté molaire estimée par RMN ¹H est supérieure à 90 %.

### Protocole 11 :

120 g de (1-(2-hydroxyéthyl)imidazolidin-2-one, 0,92 mol) sont chargés dans un réacteur de 1L muni d'un réfrigérant et purgé à l'azote. On chauffe à 90°C, et 120,4 g de chlorure de thionyle (1,02 mol) sont ajoutés sur une période de 30 minutes. On laisse réagir 2 heures à 90°C. 400 g de toluène sont ensuite ajoutés et on chauffe à reflux pendant 2 heures. Le mélange réactionnel est refroidi à température ambiante. Le précipité obtenu est filtré, lavé au toluène, puis séché sous vide. On obtient 123,2 g d'un solide blanc (point de fusion égal à 93°C, rendement massique de 90%).

### Etape b1 (non conforme à l'invention) : préparation du 2,4,6-triméthyl-3-(2-(2-oxoimidazolidin-1-yl)éthoxy)benzaldéhyde

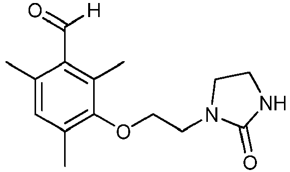

### Protocole 12 :

Ce composé peut être obtenu selon un protocole décrit dans la demande de brevet WO 2012/007684.

### Protocole 13 :

Un mélange de 3-hydroxy-2,4,6-triméthylbenzaldéhyde (25,0 g, 0,152 mol) et de K₂CO₃ (126,3 g, 0,914 mol) dans le DMF (200 mL) est agité à 30-35 °C pendant 5 à 10 minutes. Est ensuite ajoutée de la 1-(2-chloroéthyl)imidazolidin-2-one (33,9 g, 0,228 mol). La température du milieu réactionnel est portée à 90°C (T_{bain}) pendant 2,5 à 3 heures. Ensuite, une seconde portion de 1-(2-chloroéthyl)imidazolidin-2-one (22,6 g, 0,152 mol) est ajoutée et le mélange est porté à 100°C (T_{bain}) pendant 3 heures. Enfin, une troisième portion de 1-(2-chloroéthyl)imidazolidin-2-one (22,6 g, 0,152 mol.) est ajoutée et le mélange est porté à 110-115°C (T_{bain}) pendant 3 à 4 heures. Après un retour à 23°C, le milieu réactionnel est dilué par l'eau (4,0 L). La phase organique est extraite par du CH₂Cl₂ (6 fois par 200 mL). Les phases organiques rassemblées sont concentrées sous pression réduite (85 mbar, 36°C). Le résidu obtenu est repris par Et₂O (200 mL) et le mélange est agité à température ambiante pendant 2 heures. Le précipité obtenu est filtré, lavé sur le filtre par Et₂O (25 mL) et séché à température ambiante.

Un solide blanc (31,93 g, rendement massique de 76 %) est obtenu.

La pureté molaire est supérieure à 82 % (RMN ¹H).

### Protocole 14 :

A un mélange de 3-hydroxy-2,4,6-triméthylbenzaldéhyde (0,44 g, 3,3 mmol), de 1-(2-hydroxyéthyl)imidazolidin-2-one (0,64 g, 4,9 mmol) et de triphénylphosphine (1,31 g, 5,0 mmol) dans le THF (30 mL) à température ambiante, est ajoutée une solution de diisopropyl azodicarboxylate (1,01 g, 5,0 mmol) dans le THF (15 mL) pendant 5 à 10 minutes. Le milieu réactionnel est agité pendant 15 heures à température ambiante et de l'eau (15 mL) y est ensuite ajoutée. Les solvants sont concentrés sous pression réduite (28°C, 50 mbar). La phase aqueuse est extraite par l'acétate d'éthyle (trois fois par 25 mL). Les phases organiques rassemblées sont lavées par une solution aqueuse saturée en NaCl. Le solvant est concentré sous pression réduite jusqu'à environ 3 mL (30°C). Apres purification sur colonne chromatographique (Ø 1,5 cm, H 11 cm) (éluant : acétate d'éthyle : éthanol (10 : 1)). Le solvant est évaporé jusqu'à sec.

Un solide blanc (0,77 g, rendement massique de 84 %) est obtenu.

La pureté molaire est supérieure à 78 % (RMN ¹H).

### Etape b2 (non conforme à l'invention) : préparation de l'oxime de 2,4,6-triméthyl-3-(2-(2-oxoimidazolidin-1-yl)éthoxy)benzaldéhyde

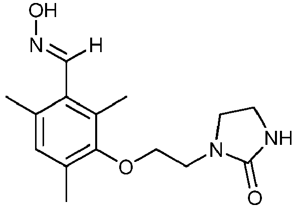

Les produits isolés à l'issue des protocoles 12, 13 et 14 sont utilisés dans les protocoles suivants (15, 16). Cette étape est non conforme à l'invention du fait de l'isolation de ces produits.

### Protocole 15 :

Ce composé peut être obtenu selon un protocole décrit dans la demande de brevet WO 2012/007684.

### Protocole 16 :

A une solution de 2,4,6-triméthyl-3-(2-(2-oxoimidazolidin-1-yl)éthoxy)benzaldéhyde (31,5 g, 0,114 mol) dans l'éthanol (300 mL) à 44°C, est ajoutée une solution d'hydroxylamine (12,0 g, 0,182 mol., 50 % dans l'eau, Aldrich) dans l'éthanol (20 mL). Le milieu réactionnel est agité pendant 4 heures à 50 °C. De l'eau (80 mL) est ajoutée à la solution. Le milieu réactionnel et concentré sous pression réduite (80 mbar, 40°C). La solution obtenue est refroidie à 15-20°C et agitée pendant 5 à 10 minutes. Le précipité obtenu est filtré, lavé sur le filtre par un mélange éthanol/eau (deux fois par 10/15 mL) et séché pendant 15-20 heures sous pression atmosphérique à température ambiante.

Un solide blanc (28,32 g, rendement massique de 85 %) est obtenu.

La pureté molaire estimée par RMN ¹H est supérieure à 87 %.

CCM : Rf = 0,58 (SiO₂ ; EtOAc : EtOH = 5 : 1 ; révélation par UV et I₂).

### Synthèse monotope en deux étapes b1 et b2 (conforme à l'invention) : préparation de l'oxime de 2,4,6-triméthyl-3-(2-(2-oxoimidazolidin-1-yl)éthoxy)benzaldéhyde

### Protocole 17 (selon l'invention) :

A un mélange de 3-hydroxy-2,4,6-triméthylbenzaldéhyde (10,0 g, 0,061 mol) et de K₂CO₃ (50,5 g, 0,365 mol) dans le DMF (100 mL) à 35°C, est ajoutée de la 1-(2-chloroéthyl)imidazolidin-2-one (13,55 g, 0,091 mol). La température du milieu réactionnel est portée à 90°C (T_{bain}) et celui-ci est agité à cette température pendant 2,5 à 3 heures. Ensuite, une seconde portion de 1-(2-chloroéthyl)imidazolidin-2-one (9,03 g, 0,061 mol) est ajoutée et le mélange est agité à 100°C (T_{bain}) pendant 2,5 heures. Enfin, la troisième portion de 1-(2-chloroethyl)imidazolidin-2-one (9,03 g, 0,061 mol., pureté technique) est ajoutée et le mélange est agité à 105°C (Tbain) pendant 4 heures.

La température du milieu réactionnel est abaissée à 43°C, puis une solution d'hydroxylamine (6,84 g, 0,104 mol, 50 % dans l'eau, Aldrich) est ajoutée. Le milieu réactionnel est agité pendant 5 heures à 50-53°C. Une seconde portion de solution d'hydroxylamine (environ 3 g, 50 % dans l'eau, Aldrich) est ajoutée. Le milieu réactionnel est agité pendant 1,5 à 2 heures à 50-53 °C. Le mélange est alors dilué par de l'eau (1,25 L). La suspension obtenue est agitée pendant 15 heures à température ambiante. Le précipité obtenu est filtré, lavé sur le filtre par l'eau (4 fois par 50 mL) et séché à température ambiante.

Un solide gris clair (12,87 g, rendement massique de 72 %) est obtenu.

La pureté molaire estimée par RMN ¹H est supérieure à 86 %.

### Protocole 18 (selon l'invention) :

Dans un réacteur de 1L, on charge 30 g de 3-hydroxy-2,4,6-triméthylbenzaldéhyde (0,18 mol) et 200 g de DMF. On ajoute 126,5 g de carbonate de potassium (0,92 mol) et on chauffe à reflux. On ajoute ensuite, par portions et sur une période de 6 heures, 67,9 g de 1-(2-chloroéthyl)imidazolidin-2-one (0,46 mol). A la fin de l'ajout, on laisse réagir pendant 2 heures à reflux. On refroidit à 50°C, puis on ajoute, sur une période de 15 minutes, 14,3 g d'une solution aqueuse d'hydroxylamine à 50% (0,22 mol). On laisse réagir pendant 2 heures à 50°C. Le mélange réactionnel est concentré jusqu'à un volume de 50 mL, puis on ajoute, à température ambiante, 500 mL d'eau. Le précipité obtenu est filtré, lavé à l'eau puis séché sous vide.

Un solide blanc-beige (44,6 g, rendement massique de 85%) avec un point de fusion de 167°C est obtenu.

### Protocole 19 (selon l'invention) :

A un mélange de 3-hydroxy-2,4,6-triméthyl-benzaldéhyde (22,8 g, 0,139 mol) et de K₂CO₃ (115,2 g, 0,833 mol) dans l'isopropanol (160 mL) à 50°C, est ajoutée de la 1-(2-chloroéthyl)imidazolidin-2-one (30,9 g, 0,208 mol). La température du milieu réactionnel est portée à 100°C (T_{bain}) et maintenue pendant 3 à 4 heures. Ensuite, une seconde portion de 1-(2-chloroéthyl)imidazolidin-2-one (20,06 g, 0,139 mol, pureté technique) est ajoutée et le mélange est agité à 100°C (T_{bain}) pendant 5 heures. Enfin, la troisième portion de 1-(2-chloroethyl)imidazolidin-2-one (20,06 g, 0,139 mol, pureté technique) est ajoutée et le mélange est agité à 100°C (Tbain) pendant 8 à 10 heures.

La température du milieu réactionnel est abaissée à température ambiante, puis de l'isopropanol (25 mL) est ajouté au milieu réactionnel. Le mélange est chauffé à 55°C et une solution d'hydroxylamine (14,68 g, 0,222 mol, 50 % dans l'eau, Aldrich) dans l'isopropanol (25 mL) est additionée. Le milieu réactionnel est agité pendant 7 heures à 50°C. Le milieu réactionnel est versé dans de l'eau (3 L). Après agitation pendant 5 heures, le précipité obtenu est filtré, lavé sur le filtre par l'eau (600 mL) et séché à température ambiante.

Un solide gris clair (30,35 g, rendement massique de 75 %) est obtenu.

La pureté molaire estimée par RMN ¹H est supérieure à 87 %.

### Protocole 20 (selon l'invention) :

Dans un réacteur de 1L, on charge 30 g de 3-hydroxy-2,4,6-triméthylbenzaldéhyde (0,18 mol) et 200 g d'isopropanol. On ajoute 126,5 g de carbonate de potassium (0,92 mol) et on chauffe à reflux. On ajoute ensuite, par portions et sur une période de 6 heures, 67,9 g de 1-(2-chloroéthyl)imidazolidin-2-one (0,46 mol). A la fin de l'ajout, on laisse réagir pendant 2 heures à reflux. On refroidit à 50°C, puis on ajoute, sur une période de 15 minutes, 14,3 g d'une solution aqueuse d'hydroxylamine à 50% (0,22 mol). On laisse réagir pendant 2 heures à 50°C. Le mélange réactionnel est concentré jusqu'à un volume de 50 mL, puis on ajoute, à température ambiante, 500 mL d'eau. Le précipité obtenu est filtré, lavé à l'eau puis séché sous vide.

Un solide blanc (43,0 g, rendement massique de 82%) avec un point de fusion de 167°C est obtenu.

Ainsi, l'oxime de 2,4,6-triméthyl-3-(2-(2-oxoimidazolidin-1-yl)éthoxy)benzaldéhyde est obtenu grâce à une synthèse monotope en deux étapes directement à partir du 3-hydroxy-2,4,6-triméthyl-benzaldéhyde.

Le procédé selon l'invention permet donc une amélioration du rendement global dudit procédé ainsi qu'une réduction des coûts d'investissement et de production.

### Exemple 3 : Synthèse de l'oxime de 3-méthoxy-4-(2-(2-oxoimidazolidin-1-yl)éthoxy)benzaldéhyde

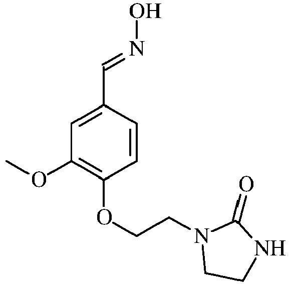

### Protocole 21 (selon l'invention) :

Dans un réacteur de 1L, on charge 30 g de vanilline (0,20 mol) et 200 g d'isopropanol. On ajoute 138,2 g de carbonate de potassium (1 mol) et on chauffe à reflux. On ajoute ensuite, par portions et sur une période de 6 heures, 89,2 g de 1-(2-chloroéthyl)imidazolidin-2-one (0,6 mol). A la fin de l'ajout, on laisse réagir pendant 2 heures à reflux. On refroidit à 50°C, puis on ajoute, sur une période de 15 minutes, 19,8 g d'une solution aqueuse d'hydroxylamine à 50% (0,30 mol). On laisse réagir pendant 2 heures à 50°C. Le mélange réactionnel est concentré jusqu'à un volume de 50 mL, puis on ajoute, à température ambiante, 500 mL d'eau. Le précipité obtenu est filtré, lavé à l'eau puis séché sous vide.

Un solide blanc (33,5 g, rendement massique de 60%) avec un point de fusion de 189°C est obtenu.

## Revendications

1. Procédé de synthèse d'un composé de formule (I) : 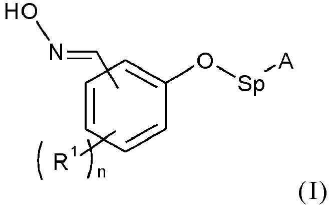 dans laquelle : 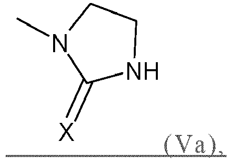
dans laquelle X est choisi parmi l'atome d'oxygène et l'atome de soufre ;
- R¹ représente un groupe alkyle en C₁-C₁₂ ou OR', R' étant un groupe alkyle en C₁-C₁₂ ;
- n est un nombre entier allant de 0 à 4 ;
- Sp représente une chaîne alkylène linéaire ou ramifiée en C₁-C₂₄, éventuellement interrompue par un ou plusieurs atomes d'azote ou d'oxygène,
comprenant deux étapes successives (b1) et (b2) selon une synthèse monotope :
- (b1) la réaction d'un composé de formule (II) : 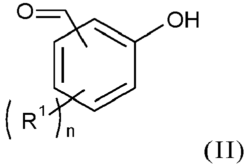
avec un composé de formule (III) : Z-Sp-A (III) dans laquelle Z représente un groupe nucléofuge ;
en présence :
- d'au moins un solvant polaire ;
- d'au moins une base choisie parmi les carbonates alcalins ;
à une température T₁ allant de 70 à 150°C,
pour former un éther ;
- (b2) la réaction dudit éther avec une solution aqueuse d'hydroxylamine à une température T₂ allant de 30 à 70°C.

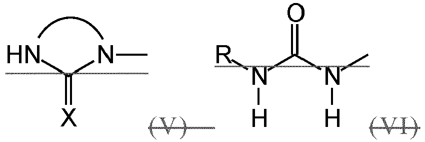

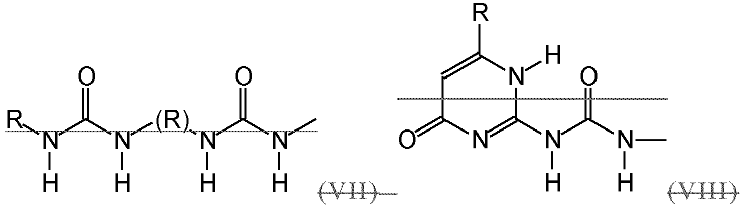

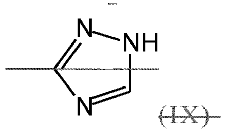

2. Procédé selon la revendication précédente, dans lequel 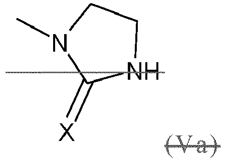 X est l'atome d'oxygène.

3. Procédé selon la revendications- 1 ou 2 dans lequel le groupe Z est choisi parmi le chlore, le brome, l'iode, le groupe mésylate, le groupe tosylate, le groupe acétate et le groupe trifluorométhylsulfonate.

4. Procédé selon l'une quelconque des revendications précédentes, dans lequel le solvant polaire est un solvant polaire miscible dans l'eau, préférentiellement un solvant protique.

5. Procédé selon l'une quelconque des revendications précédentes, dans lequel ledit procédé comprend une étape (a1) de fabrication du composé de formule (II), préalable à l'étape (b1), par mise en réaction d'un composé de formule (X) : 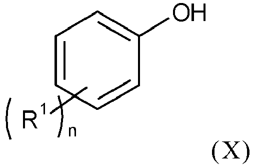 avec un agent de formylation formé in situ ou non et au moins un acide de Lewis, en présence d'au moins un solvant organique.

6. Procédé selon la revendication précédente, dans lequel l'acide de Lewis est choisi parmi TiCl₄ et SnCl₄, préférentiellement TiCl₄.

7. Procédé selon l'une quelconque des revendications 5 à 6, dans lequel le solvant organique est un solvant chloré, préférentiellement choisi parmi le dichlorométhane, le chloroforme et le 1,2-dichloroéthane, plus préférentiellement le dichlorométhane.

8. Procédé selon l'une quelconque des revendications 5 à 7, dans lequel l'agent de formylation est choisi parmi le dichlorométhyl-méthyléther et le dichlorométhyl-éthyléther, préférentiellement le dichlorométhyl-méthyléther.

9. Procédé selon l'une quelconque des revendications 5 à 8, dans lequel l'agent formé in situ est obtenu par réaction du formiate de méthyle ou d'éthyle avec un agent de chloration, préférentiellement le pentachlorure de phosphore.

10. Procédé selon l'une quelconque des revendications précédentes, dans lequel ledit procédé comprend une étape (a2) de fabrication du composé de formule (III), préalable à l'étape (b1), par mise en réaction d'un composé de formule (XI) : HO-Sp-A (XI) avec un agent permettant la formation du groupe nucléofuge Z.

11. Procédé selon la revendication précédente, dans lequel ledit agent est le chlorure de thionyle.

## Patentansprüche

1. Verfahren zur Synthese einer Verbindung der Formel (I) : 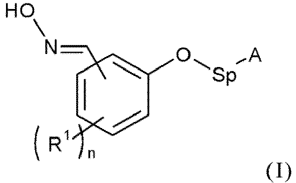 in der:
- A die Gruppe der Formel (Va) ist: 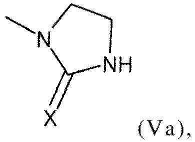 in der X aus dem Sauerstoffatom und dem Schwefelatom ausgewählt ist;
- R¹ für eine C₁-C₁₂-Alkylgruppe oder OR' steht, wobei R' eine C₁-C₁₂-Alkylgruppe ist;
- n eine ganze Zahl im Bereich von 0 bis 4 ist;
- Sp für eine lineare oder verzweigte C₁-C₂₄-Alkylenkette, die gegebenenfalls durch ein oder mehrere Stickstoffoder Sauerstoffatome unterbrochen ist, steht,
umfassend die beiden aufeinanderfolgenden Schritte (b1) und (b2) gemäß einer Eintopfsynthese:
- (b1) die Umsetzung einer Verbindung der Formel (II): 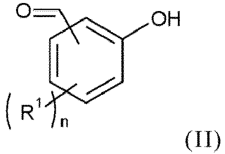
mit einer Verbindung der Formel (III): Z-Sp-A (III), in der Z für eine nukleofuge Gruppe steht;
in Gegenwart von:
- mindestens einem polaren Lösungsmittel;
- mindestens einer aus Alkalicarbonaten ausgewählten Base;
bei einer Temperatur T₁ im Bereich von 70 bis 150 °C zur Bildung eines Ethers;
- (b2) die Umsetzung des Ethers mit einer wässrigen Lösung von Hydroxylamin bei einer Temperatur T₂ im Bereich von 30 bis 70 °C.

2. Verfahren nach dem vorhergehenden Anspruch, wobei X für das Sauerstoffatom steht.

3. Verfahren nach Anspruch 1 oder 2, wobei die Gruppe Z aus Chlor, Brom, Iod, der Mesylatgruppe, der Tosylatgruppe, der Acetatgruppe und der Trifluormethylsulfonatgruppe ausgewählt ist.

4. Verfahren nach einem der vorhergehenden Ansprüche, wobei es sich bei dem polaren Lösungsmittel um ein mit Wasser mischbares polares Lösungsmittel, bevorzugt ein protisches Lösungsmittel, handelt.

5. Verfahren nach einem der vorhergehenden Ansprüche, wobei das Verfahren einen Schritt (a1) der Herstellung einer Verbindung der Formel (II) vor Schritt (b1) durch Umsetzen einer Verbindung der Formel (X): 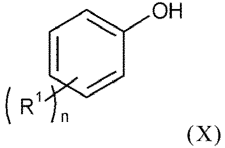 mit einem gegebenenfalls in situ gebildeten Formylierungsmittel und mindestens einer Lewis-Säure in Gegenwart mindestens eines organischen Lösungsmittels umfasst.

6. Verfahren nach dem vorhergehenden Anspruch, wobei die Lewis-Säure aus TiCl₄ und SnCl₄, bevorzugt TiCl₄, ausgewählt wird.

7. Verfahren nach einem der Ansprüche 5 bis 6, wobei es sich bei dem organischen Lösungsmittel um ein chloriertes Lösungsmittel handelt, bevorzugt ausgewählt aus Dichlormethan, Chloroform und 1,2-Dichlorethan, bevorzugt Dichlormethan.

8. Verfahren nach einem der Ansprüche 5 bis 7, wobei das Formylierungsmittel aus Dichlormethyl(methyl)ether und Dichlormethyl(ethyl)ether, vorzugsweise Dichlormethyl(methyl)ether, ausgewählt wird.

9. Verfahren nach einem der Ansprüche 5 bis 8, wobei das in situ gebildete Mittel durch Umsetzung von Ameisensäuremethylester oder Ameisensäureethylester mit einem Chlorierungsmittel, bevorzugt Phosphorpentachlorid, erhalten wird.

10. Verfahren nach einem der vorhergehenden Ansprüche, wobei das Verfahren einen Schritt (a2) der Herstellung der Verbindung der Formel (III) vor Schritt (b1) durch Umsetzen einer Verbindung der Formel (XI): HO-Sp-A (XI) mit einem Mittel, das die Bildung der nukleofugen Gruppe Z ermöglicht, umfasst.

11. Verfahren nach dem vorhergehenden Anspruch, wobei es sich bei dem Mittel um Thionylchlorid handelt.

## Claims

1. Process for the synthesis of a compound of formula (I): 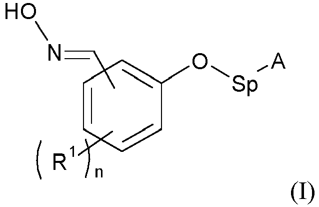 in which:
- A is the group of formula (Va): 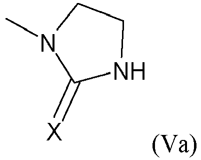 in which X is chosen from the oxygen atom and the sulfur atom;
- R¹ represents a C₁-C₁₂ alkyl group, or OR', R' being a C₁-C₁₂ alkyl group;
- n is an integer ranging from 0 to 4;
- Sp represents a linear or branched C₁-C₂₄ alkylene chain, optionally interrupted by one or more nitrogen or oxygen atoms;
comprising two successive stages (b1) and (b2) according to a one-pot synthesis:
- (b1) the reaction of a compound of formula (II): 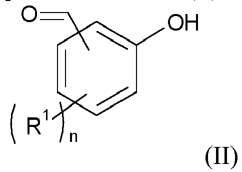
with a compound of formula (III): Z-Sp-A (III), in which Z represents a nucleofuge group;
in the presence:
- of at least one polar solvent;
- of at least one base chosen among alkali metal carbonates;
at a temperature T₁ ranging from 70 to 150°C;
in order to form an ether;
- (b2) the reaction of the said ether with an aqueous hydroxylamine solution at a temperature T₂ ranging from 30 to 70°C.

2. Process according to the preceding claim, in which X is the oxygen atom.

3. Process according to either one of Claims 1 and 2, in which the Z group is chosen from chlorine, bromine, iodine, the mesylate group, the tosylate group, the acetate group and the trifluoromethylsulfonate group.

4. Process according to any one of the preceding claims, in which the polar solvent is a water-miscible polar solvent, preferably a protic solvent.

5. Process according to any one of the preceding claims, in which the said process comprises a stage (a1) of manufacture of the compound of formula (II), prior to stage (b1), by reacting a compound of formula (X): 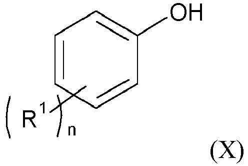 with a formylating agent, formed or not formed in situ, and at least one Lewis acid in the presence of at least one organic solvent.

6. Process according to the preceding claim, in which the Lewis acid is chosen from TiCl₄ and SnCl₄, preferably TiCl₄.

7. Process according to either one of Claims 5 and 6, in which the organic solvent is a chlorinated solvent, preferably chosen from dichloromethane, chloroform and 1,2-dichloroethane, more preferably dichloromethane.

8. Process according to any one of Claims 5 to 7, in which the formylating agent is chosen from dichloromethyl methyl ether and dichloromethyl ethyl ether, preferably dichloromethyl methyl ether.

9. Process according to any one of Claims 5 to 8, in which the agent formed in situ is obtained by reaction of methyl or ethyl formate with a chlorinating agent, preferably phosphorus pentachloride.

10. Process according to any one of the preceding claims, in which the said process comprises a stage (a2) of manufacture of the compound of formula (III), prior to stage (b1), by reacting a compound of formula (XI): HO-Sp-A (XI), with an agent which makes possible the formation of the nucleofuge group Z.

11. Process according to the preceding claim, in which the said agent is thionyl chloride.
